# EUROPÄISCHE PATENTSCHRIFT

(11) **EP 2 782 888 B1**
(45) Veröffentlichungstag und Bekanntmachung des Hinweises auf die Patenterteilung: **03.01.2018**
(21) Anmeldenummer: 12778343.9
(22) Anmeldetag: 25.10.2012
(51) Int. Cl.: C07C 2/06, C07C 7/04, C07C 7/177

(54) **VERFAHREN ZU HERSTELLUNG VON OLIGOMEREN DES BUTEN**
PROCESS FOR PRODUCING BUTENE OLIGOMERS
PROCÉDÉ DE PRODUCTION D'OLIGOMÈRES DE BUTÈNE

(30) Priorität: 21.11.2011 EP 11189917
(43) Veröffentlichungstag der Anmeldung: 01.10.2014
(73) Patentinhaber: BASF SE, 67056 Ludwigshafen am Rhein (DE)
(72) Erfinder: CRONE, Sven, 67117 Limburgerhof (DE); RYLL, Oliver, 67480 Edenkoben (DE); BLUM, Till, Singapore 238713 (SG); WECK, Alexander, 67251 Freinsheim (DE); PAPP, Rainer, 67346 Speyer (DE); KROKOSZINSKI, Roland, 67273 Weisenheim a.Berg (DE); BLANKERTZ, Heinrich-Josef, 67147 Forst (DE)
(74) Vertreter: BASF IP Association
(86) Internationale Anmeldenummer: PCT/EP2012/071106
(87) Internationale Veröffentlichungsnummer: WO 2013/075905

(56) Entgegenhaltungen:
- WO-A1-95/14647
- DE-A1- 19 922 038

## Beschreibung

Die vorliegende Anmeldung schließt durch Verweis die am 21. November 2011 einreichte vorläufige US-Anmeldung 61/561951 ein.

Die Erfindung betrifft ein Verfahren zur Herstellung von Oligomeren durch kontinuierliche Oligomerisierung von Butenen, welches dadurch gekennzeichnet ist, dass
a) ein Ausgangsstrom, welcher 1-Buten und 2-Buten in einer Gesamtkonzentration von 10 bis 70 Gew. % und 10 bis 60 Gew. % iso-Butan enthält, verwendet wird (im Nachfolgenden Ausgangsstrom 1 genannt)
b) dieser Ausgangsstrom 1 umgesetzt wird bis mehr als 60 Gew. % des im Ausgangsstrom 1 enthaltenen 1-Butens, aber weniger als 50 Gew. % des im Ausgangsstrom 1 enthaltenen 2-Butens in Oligomere überführt sind
c) die in b) erhaltenen Oligomere abgetrennt und gegebenenfalls einer weiteren Aufarbeitung zugeführt werden und der verbleibende Reststrom einer destillativen Aufarbeitung zugeführt wird
d) aus dem Reststrom iso-Butan destillativ abgetrennt wird
e) der nach der destillativen Aufarbeitung d) erhaltene, an iso-Butan abgereicherte Strom (im Nachfolgenden Ausgangsstrom 2 genannt) zu Oligomeren umgesetzt wird.

Oligomere des Butens, wie Octene und Dodecene, haben eine hohe Bedeutung als Ausgangsstoffe für weitere chemische Synthesen und als Bestandteile von Treibstoffen wie Benzin, Diesel oder Kerosin. So werden diese Oligomere z. B. durch Hydroformylierung und anschließende Hydrierung in Alkohole überführt. Die erhaltenen Alkohole eignen sich zur Herstellung von Weichmachern und Tensiden.

Verfahren zur Oligomerisierung von Butenen sind bekannt. In WO 00/69795 wird ein Oligomerisierungsverfahren beschrieben, bei dem die Oligomerisierung in mehreren Stufen, das heißt in mehreren nacheinander folgenden adiabatisch betriebenen Reaktionszonen an einem heterogenen, Nickel enthaltenden Oligomerisierungskatalysator erfolgt.

Die für eine Oligomerisierung verwendeten Stoffströme sollten einen möglichst hohen Gehalt an linearen Butenen aufweisen. Ein Gehalt an inerten Butanen verringert die Raumzeit Ausbeute.

Gemäß EP-A 1724 249 werden n- und iso-Butan daher vor der Durchführung der Oligomerisierung durch ein mehrstufiges Verfahren abgetrennt. Das mehrstufige Verfahren umfasst eine Extraktivdestillation, eine destillative Aufarbeitung des dabei erhaltenen Sumpfes und Waschen der Kopf-fraktion.

Die Auftrennung von Butenen und Butanen durch Extraktivdestillation ist z.B. auch in EP-A 149145 beschrieben.

Für die Oligomerisierung von Butenen können sogenannte Raffinat-2-Ströme verwendet werden, die aus Steam Crackern oder Raffinerien entnommen werden können oder auch als Endprodukte anderer chemischer Verfahren, Dehydrierungen oder MTO-Verfahren (methanol to olefines) zur Verfügung stehen. Im Wesentlichen handelt es sich bei Raffinat-2-Strömen um C4-Ströme.

Aus derartigen Raffinat-2-Strömen wurden Butadien und Isobuten im Allgemeinen bereits durch vorangegangene Prozessschritte größtenteils entfernt. Bekannte Methoden zur Abtrennung von Butadien sind Anlagen zur Extraktion von Butadien oder die Selektivhydrierung von Butadien zu Butenen.

Raffinat-2-Ströme aus Raffinerien enthalten im Allgemeinen deutlich weiniger für die Oligomerisierung geeignete 1- und 2-Butene als solche aus Steam Crackern.

Bei einem geringen Gehalt an Butenen in den Raffinat-2-Stroemen müssen die Reaktoren und der Aufarbeitungsteil der Oligomerisierung entsprechend groß ausgelegt sein, um eine zufriedenstellende Ausbeute an Oligomeren und effektive Nutzung der im Raffinat enthaltenden linearen Butene zu erreichen.

Gewünscht ist daher ein einfaches und effektives Verfahren, bei dem auch Ströme mit einem geringen Gehalt an linearen Butenen, z.B. Raffinat-2-Ströme aus Raffinerien, mit hoher Raum-Zeit Ausbeute oligomerisiert werden können. Insbesondere sollen dazu die zur Verfügung stehenden linearen Butene sowie die Kapazität der Reaktoren und Aufarbeitungsteile möglichst optimal genutzt werden können.

Aufgabe der vorliegenden Erfindung war daher, ein entsprechendes Verfahren zur Verfügung zu stellen.

Demgemäß wurde das eingangs definierte Verfahren gefunden.

Nachstehend werden Ausführungsformen des erfindungsgemäßen Verfahrens beschrieben.

### Zu a)

Ein geeigneter Ausgangsstrom 1 enthält überwiegend Kohlenwasserstoffe mit 4 C- Atomen (C4-Kohlenwasserstoffe), insbesondere besteht er zu mehr als 85 Gew. %, bevorzugt zu mehr als 95 Gew. besonders bevorzugt zu mehr als 99 Gew. % aus C4-Kohlenwasserstoffen.

Als Ströme mit einem derartigen C4 Kohlenwasserstoffgehalt bieten sich z.B. sogenannte Raffinat-2-Ströme an, die aus Steam Crackern oder Raffinerien entnommen werden können oder auch als Endprodukte anderer chemischer Verfahren zur Verfügung stehen.

Vorzugsweise enthält Ausgangstrom 1 kein oder nur geringe Mengen an Butadien und Isobuten. Soweit ursprünglich vorhanden, werden diese im Allgemeinen durch vorangegangene Prozessschritte größtenteils entfernt. Bekannte Methoden zur Abtrennung von Butadien sind Anlagen zur Extraktion von Butadien oder die Selektivhydrierung von Butadien zu Butenen.

Isobuten kann z. B. durch Herstellung von Methyltertiärbutylether (MTBE) oder Isobutyltertiärbutylether (IBTBE) mit anschließender Rückspaltung abgetrennt werden.

Nachstehende Gewichtsangaben sind auf das Gesamtgewicht des Ausgangsstrom 1 bezogen.

Der Ausgangstrom 1 enthält vorzugsweise weniger als 3 Gew. %, insbesondere weniger als 1 Gew.%, besonders bevorzugt weniger als 0,3 Gew. % Butadien.

Der Ausgangstrom 1 enthält vorzugsweise weniger als 3 Gew. %, insbesondere weniger als 1 Gew. %, besonders bevorzugt weniger als 0,3 bzw. weniger als 0,1 Gew. % Isobuten.

Ausgangsstrom 1 enthält 1-Buten und 2-Buten in einer Gesamtmenge von 10 bis 70 Gew. %.

1-Buten und 2-Buten sind lineare Butene mit der Doppelbindung in 1,2- bzw. in 2,3- Stellung.

Vorzugsweise enthält Ausgangsstrom 1-Buten und 2-Buten in einer Gesamtkonzentration von 20 bis 60 Gew. %, besonders bevorzugt in einer Gesamtkonzentration von 30 bis 60 Gew. %, ganz besonders bevorzugt in einer Gesamtkonzentration von 40 bis 60 Gew. %.

Ausgangsstrom 1 enthält sowohl 1-Buten als auch 2-Buten.

Vorzugsweise enthält Ausgangsstrom 1
1 bis 30 Gew. % 1-Buten und 1 bis 40 Gew. % 2-Buten,
   besonders bevorzugt
5 bis 30 Gew. % 1-Buten und 5 bis 40 Gew. % 2-Buten,
   wobei die Gesamtmenge der beiden Butene 10 bis 70 Gew. % bzw. 20 bis 60 Gew. % beträgt.

Ganz besonders bevorzugt beträgt der Gehalt an 1-Buten 5 bis 30 Gew. % und an 2-Buten 20 bis 40 Gew. %, wobei der Gesamtgehalt der beiden Butene 40 bis 60 Gew. % beträgt.

Ausgangsstrom 1 enthält 10 bis 60 Gew. % iso-Butan, insbesondere 15 bis 50 Gew. % und besonders bevorzugt 20 bis 50 Gew. % iso-Butan. In einer ganz besonders bevorzugten Ausführungsform enthält Ausgangstrom 1 iso-Butan in einer Menge von 30 bis 50 Gew. %.

Neben 1-Buten, 2-Buten, iso-Butan kann der Ausgangsstrom 1 n-Butan und weitere Bestandteile, wozu z. B. ggf. geringe Mengen an Butadien, Isobuten (siehe oben) oder Verunreinigungen zählen.

Insbesondere enthält Ausgangsstrom 1 auch n-Butan, z. B. in einer Menge von 0 bis 60 Gew. %, insbesondere 5 bis 60 Gew. %, vorzugsweise 5 bis 30 Gew. %

Bevorzugt besteht Ausgangsstrom 1 insgesamt aus
1 bis 30 Gew. % 1-Buten
1 bis 35 Gew. % 2-Buten
10 bis 60 Gew. % iso-Butan
0 bis 60 Gew. % n-Butan
0 bis 10 Gew. % weitere Bestandteile

Besonders bevorzugt besteht Ausgangsstrom 1 insgesamt aus
5 bis 30 Gew. % 1-Buten
5 bis 35 Gew. % 2-Buten
15 bis 60 Gew. % iso-Butan
5 bis 60 Gew. % n-Butan
0 bis 5 Gew. % weitere Bestandteile

Ganz besonders bevorzugt besteht Ausgangsstrom 1 insgesamt aus
5 bis 30 Gew. % 1-Buten
20 bis 40 Gew. % 2-Buten
20 bis 50 Gew. % iso-Butan
5 bis 30 Gew. % n-Butan
0 bis 2 Gew. % weitere Bestandteile

In einer besonderen Ausführungsform besteht Ausgangsstrom 1 insgesamt aus
10 bis 30 Gew. % 1-Buten
20 bis 40 Gew. % 2-Buten
30 bis 50 Gew. % iso-Butan
5 bis 20 Gew. % n-Butan
0 bis 2 Gew. % weitere Bestandteile

### Zu b)

In Verfahrensschritt b) erfolgt eine Oligomerisierung. Unter dem Begriff Oligomerisierung wird hier auch eine Dimerisierung verstanden. Bei der Oligomerisierung werden 1-Buten und 2-Buten zu Octenen (Dimerisierung), oder weiter zu Dodecene (Trimerisierung) oder höheren Oligomeren (C16 oder mehr, zusammenfassend kurz C16+) umgesetzt.

Als Octene entstehen bei der Dimerisierung nahezu ausschließlich iso-Octene, das sind z.B. einfach- oder zweifach- oder dreifach verzweigte C8-Olefine (wie z.B.3-Methyl-Hepten oder 3,4-Dimethyl-Hexen). Entsprechend entstehen als Trimer nahezu ausschließlich iso-Dodecene.

Daher soll der Begriff Octen in dieser Patentanmeldung Octene jeglicher Art umfassen und insbesondere für Octengemische, stehen, wie sie bei der Oligomersisierung entstehenden, also für Gemische im Wesentlichen von verschiedenen iso-Octenen.

Entsprechend soll der Begriff Dodecen in dieser Patentanmeldung Dodecene jeglicher Art umfassen und insbesondere für Dodecengemische, stehen, wie sie bei der Oligomersisierung entstehenden, also für Gemische im Wesentlichen von verschiedenen iso-Dodecenen.

Verfahren zur Oligomerisierung sind bekannt und z.B. in EP-A 1724 249 oder WO 00/69795 beschrieben.

Bevorzugt erfolgt die Oligomerisierung in Gegenwart eines heterogenen Katalysators.

Als heterogene Katalysatoren kommen z.B. sauren Katalysatoren in Betracht.

Bevorzugt sind Nickel enthaltende heterogene Katalysatoren, besonders bevorzugt ist Nickel in Form von Nickeloxid (NiO). Neben NiO kann der heterogene Katalysator weitere Bestandteile enthalten, z.B. SiO₂, TiO₂, ZrO₂ oder Al₂O₃ als Trägermaterial.

Die Oligomerisierung in Verfahrensschritt b) kann in einer Reaktionszone oder in mehreren parallel oder in Reihe geschalteten Reaktionszonen erfolgen.

Vorzugsweise erfolgt die Oligomerisierung bei Temperaturen von 30 bis 180°C, besonders bevorzugt bei Temperaturen von 30 bis 140°C.

Vorzugsweise erfolgt die Oligomerisierung bei Drucken von 10 bis 300 bar besonders bevorzugt bei Drucken von 15 bis 100 bar.

Als Reaktoren sind z.B. mit dem Katalysator beschickte zylindrische Reaktoren geeignet. Diese werden von dem vorzugsweise flüssigen Ausgangsgemisch 1 von oben nach unten oder umgekehrt durchströmt. Bei mehreren Reaktionszonen werden entsprechend in Reihe oder parallel geschaltete Reaktoren verwendet oder ein Reaktor der entsprechend in mehrere Reaktionszonen unterteilt ist.

Vorzugsweise erfolgt die Oligomerisierung in Verfahrensschritt b) in nur einer Reaktionszone.

Bei der Oligomerisierung werden zunächst 1-Butene umgesetzt, diese haben eine höhere Reaktivität.

Ausgangsstrom 1 wird in Verfahrensschritt b) umgesetzt bis mehr als 60 Gew. % aller im Ausgangsstrom 1 enthaltenen 1-Butene, aber weniger als 50 Gew. % der im Ausgangsstrom 1 enthaltenen 2-Butene in Oligomere überführt sind.

Vorzugsweise wird Ausgangsstrom 1 in Verfahrensschritt b) umgesetzt bis mehr als 80 Gew. % aller im Ausgangsstrom 1 enthaltenen 1-Butene, aber weniger als 50 Gew. % der im Ausgangsstrom 1 enthaltenen 2-Butene in Oligomere überführt sind.
Besonders bevorzugt wird Ausgangsstrom 1 in Verfahrensschritt b) umgesetzt bis mehr als 85 Gew. % aller im Ausgangsstrom 1 enthaltenen 1-Butene, aber weniger als 35 Gew. % der im Ausgangsstrom 1 enthaltenen 2-Butene in Oligomere überführt sind.

Ganz besonders bevorzugt wird Ausgangsstrom 1 in Verfahrensschritt b) umgesetzt bis mehr als 85 Gew. % aller im Ausgangsstrom 1 enthaltenen 1-Butene, aber weniger als 25 Gew. % der im Ausgangsstrom 1 enthaltenen 2-Butene in Oligomere überführt sind.

Der erreichte Umsatz von 1-Buten und 2- Buten kann leicht bestimmt werden durch gaschromatographische Quantifizierung der verbliebenen Menge an 1- und 2-Buten.

### Zu c)

Verfahrensschritt c) ist die Abtrennung der in b) gebildeten Oligomeren.
Die Oligomeren werden vorzugsweise abgetrennt durch Destillation, wobei die Oligomere mit dem Sumpf ausgetragen werden und die C4-Fraktion über Kopf abgezogen wird.

Die Oligomeren werden einer weiteren Aufarbeitung zugeführt. Dabei erfolgt insbesondere eine Auftrennung in die Fraktionen Octen, Dodecen und C16+. Die Auftrennung kann in bekannter Weise durch Destillation erfolgen.

Der verbleibende Reststrom wird einer destillativen Aufarbeitung zugeführt, welche unter Verfahrenschritt d) beschrieben wird.

### Zu d)

In Verfahrensschritt d) wird iso-Butan aus dem Reststrom destillativ abgetrennt.

Die Gesamtmenge des in c) erhaltenen und nun in d) zu destillierenden Reststroms beträgt vorzugsweise 5 bis 95 Gew. %, insbesondere 30 bis 95 Gew. %, besonders bevorzugt 50 bis 90 Gew. % und ganz besonders bevorzugt 60 bis 85 Gew. % des Ausgangsstroms 1.

Der Reststrom besteht insbesondere insgesamt aus
10 bis 60 Gew. % 2-Buten
1 bis 30 Gew. % 1-Buten
20 bis 70 Gew. % iso-Butan
1 bis 40 Gew. % n-Butan
0 bis 10 Gew. % sonstigen Bestandteilen

Der Reststrom besteht besonders bevorzugt insgesamt aus
20 bis 50 Gew. % 2-Buten
1 bis 20 Gew. % 1-Buten
30 bis 60 Gew. % iso-Butan
1 bis 30 Gew. % n-Butan
0 bis 5 Gew. % sonstigen Bestandteilen

Der Reststrom besteht ganz besonders bevorzugt aus
30 bis 40 Gew. % 2-Buten
1 bis 10 Gew. % 1-Buten
40 bis 55 Gew. % iso-Butan
10 bis 20 Gew. % n-Butan
0 bis 5 Gew. % sonstigen Bestandteilen

Die Destillation in Verfahrensschritt d) kann nach bekannten Verfahren durchgeführt werden.

Zur Abtrennung des iso-Butans ist es nicht notwendig eine Extraktivdestillation durchzuführen. In einer bevorzugten Ausführungsform handelt es sich daher nicht um eine Extraktivdestillation und es wird kein Extraktionsmittel zugesetzt. Vorzugsweise wird in Verfahrensschritt d) keinerlei Lösemittel zugesetzt.

Die Destillation wird vorzugsweise bei Kopf-Temperaturen von 50 bis 90°C, besonders bevorzugt von 60 bis 80°C und ganz besonders bevorzugt von 65 bis 75°C, und bei Sumpf-Temperaturen von 60 bis 110°C, besonders bevorzugt von 70 bis 100°C und ganz besonders bevorzugt von 80 bis 90°C durchgeführt.

Die Destillation wird vorzugsweise bei Sumpf-Drücken von 8 bis 15 bar, besonders bevorzugt von 9 bis 13 bar und ganz besonders bevorzugt von 10 bis 12 bar durchgeführt. Der Druckverlust über die ganze Kolonne kann dabei zum Beispiel 0.1 - 0.5 bar betragen.

Als Destillationskolonnen geeignet sind sowohl Füllkörper-kolonnen als auch Kolonnen mit eingebauten Kolonnenböden (Bodenkolonnen). Geeignet sind auch Kolonnen, die sowohl Füllkörper als auch Böden enthalten, z.B. Füllkörperschüttungen in Teilen der Kolonnen und entsprechende Einbauten (Stahlbleche) in anderen Teilen. Bevorzugt ist eine Bodenkolonne. Die Bodenkolonne kann z. B: 40 bis 150, insbesondere 80 bis 120 Böden enthalten

Die Destillationskolonne kann vorzugsweise mindestens 5, insbesondere mindestens 10 theoretische Böden enthalten Die Anzahl der theoretischen Böden kann z. B. insgesamt 10 bis 100, insbesondere -20 bis 100 bzw. 30 bis 100 betragen. In einer besonderen Ausführungsform enthält die Destillationskolonne insgesamt 40-70 theoretische Böden. In einer bevorzugten Ausführungsform unterteilt sich die Kolonne in einen Abtriebsteil und einen Verstärkungsteil; der Abtriebsteil kann z.B. 25-40 theoretische Boeden und der Verstärkungsteil 15-30 theoretische Boeden enthalten.

Durch die Destillation werden vorzugsweise mehr als 60 Gew. %, insbesondere mehr als 70 Gew. %, besonders bevorzugt mehr als 75 Gew. % und ganz besonders bevorzugt mehr als 80 Gew. % des im Reststrom enthaltenen iso-Butans abgetrennt.

Das abgetrennte iso-Butan und ggf. weitere abgetrennte Verbindungen (im Nachfolgenden zusammenfassend Abfallstrom genannt) werden vorzugsweise kondensiert und können für sonstige Zwecke verwendet werden.

Der Abfallstrom besteht insbesondere aus
50 bis 100 Gew. % iso-Butan
0 bis 20 Gew. % n-Butan
0 bis 20 Gew. % 2-Buten
0 bis 20 Gew. % 1-Buten
0 bis 5 Gew. % sonstigen Bestandteilen

Der Abfallstrom besteht besonders bevorzugt aus
70 bis 95 Gew. % iso-Butan
0 bis 10 Gew. % n-Butan
0 bis 10 Gew. % 2-Buten
0 bis 10 Gew. % 1-Buten
0 bis 5 Gew. % sonstigen Bestandteilen

Der Abfallstrom besteht ganz besonders bevorzugt aus
80 bis 95 Gew. % iso-Butan
0 bis 8 Gew. % n-Butan
0 bis 8 Gew. % 2-Buten
0 bis 8 Gew. % 1-Buten
0 bis 1 Gew. % sonstigen Bestandteilen

Zurück bleibt ein an 2-Buten reiches Gemisch, welches vorzugsweise am Boden der Kolonnen entnommen werden kann. Dieses Gemisch wird als Ausgangsstrom 2 erneut einer Oligomerisierung zugeführt.

In einer besonderen Ausführungsform können Verfahrensschritte c) und d) in einer gemeinsamen Destillationskolonne durchgeführt werden. Dabei werden die Oligomeren (Octen und höhere Oligomere) als Sumpfaustrag erhalten, Ausgangsstrom 2 (Buten-reicher Strom) wird als Seitenabzug abgeführt und am Kopf wird der verbleibende AbfallstromButan-reicher Strom) abgenommen.

### Zu e)

Der in d) erhaltenen Ausgangsstrom 2 wird in Verfahrensschritt e) wiederum zu Oligomeren umgesetzt.

Ausgangsstrom 2 besteht vorzugsweise zu mehr als 40 Gew. %, insbesondere zu mehr als 50 Gew. % und ganz besonders bevorzugt zu mehr als 60 Gew.% aus 2-Buten.

Ausgangsstrom 2 besteht vorzugsweise insgesamt aus
40 bis 80 Gew. % 2-Buten
0 bis 20 Gew. % 1-Buten
1 bis 20 Gew. % iso-Butan
5 bis 50 Gew. % n-Butan
0 bis 10 Gew. % sonstigen Bestandteilen

Ausgangsstrom 2 besteht besonders bevorzugt insgesamt aus
50 bis 80 Gew. % 2-Buten
0 bis 10 Gew. % 1-Buten
1 bis 10 Gew. % iso-Butan
10 bis 40 Gew. % n-Butan
0 bis 5 Gew. % sonstigen Bestandteilen

Ausgangsstrom 2 besteht ganz besonders bevorzugt insgesamt aus
60 bis 70 Gew. % 2-Buten
0 bis 5 Gew. % 1-Buten
1 bis 10 Gew. % iso-Butan
20 bis 40 Gew. % n-Butan
0 bis 2 Gew. % sonstigen Bestandteilen

Zur Durchführung der Oligomerisierung gelten hier die obigen Ausführungen unter b) entsprechend.

Auch zur Aufarbeitung der erhaltenen Oligomeren gelten die obigen Ausführungen entsprechend.

In einer bevorzugten Ausführungsform erfolgt die Aufarbeitung der in b) erhaltenen Oligomeren gemeinsam mit den in e) erhaltenen Oligomeren. In einer bevorzugten Ausführungsform werden die Oligomeren aus b) und aus e) einer gemeinsamen Destillation zur Auftrennung in Octen, Dodecen und C16+ zugeführt.

Bei den in Verfahrensschritten b) und e) insgesamt erhaltenen Oligomeren handelt es sich vorzugsweise um ein Gemisch aus
70 bis 100 Gew.%, insbesondere um 75 bis 90 Gew. % Octen
0 bis 30 Gew. %, insbesondere 5 bis 20 Gew. % Dodecen
0 bis 20 Gew. %, insbesondere 1 bis 10 Gew. % C16+
bezogen auf die Gesamtmenge aller in b) und e) entstandenen Oligomeren.

Bei der Oligomerisisierung unter e) fällt als Rückstand ein an n-Butan reiches Gemisch an, welches 1-Buten und 2-Buten vorzugsweise allenfalls in einer Gesamtmenge von weniger als 25 Gew. %, besonders bevorzugt von weniger als 20 Gew. % enthält. Dieses Gemisch kann für andere Zwecke verwendet werden.

Das erfindungsgemäße Verfahren ist ein kontinuierliches Verfahren, vorzugsweise werden daher alle vorstehenden Verfahrensschritte kontinuierlich betrieben.

Es ist ein Vorteil des erfindungsgemäßen Verfahrens, dass Ströme mit einem geringen Gehalt an linearen Butenen (1-Buten und 2-Buten) z.B. Raffinat-2-Ströme aus Raffinerien, mit hoher Raum-Zeit Ausbeute oligomerisiert werden können. Dazu kann die Kapazität der Reaktoren und Aufarbeitungsteile optimal genutzt werden.

Durch das erfindungsgemäßen Verfahren können die im Ausgangstrom 1 enthaltenen 1- und 2-Butene insgesamt zu mehr als 85 Gew. %, insbesondere zu mehr als 90 Gew. % in Oligomere überführt werden.

Es ist ein weiterer Vorteil des erfindungsgemäßen Verfahrens, dass es auch leicht in bestehenden Oligomerisierungsanlagen durchgeführt werden kann. Dazu sind im Allgemeinen nur wenige apparative Änderungen notwendig. Das erfindungsgemäße Verfahren erlaubt daher eine flexible Anpassung an verschiedene Rohstoffquellen. Insbesondere ist bei einem Wechsel von C4-Quellen mit einem hohen Gehalt an Butenen zu solchen mit einem geringen Gehalt an Butenen eine apparative Anpassung und Durchführung des erfindungsgemäßen Verfahrens leicht möglich.

### Beispiele

Es wurden zwei Simulationen einer Buten-Dimerisierung durchgeführt. Bei ansonsten identischen Rahmenbedingungen, das heißt bei gleichem Ausgangsstrom, identische Vorrichtungen (Reaktoren und Vorrichtungen des Aufarbeitungsteils) wurde die Zusammensetzung aller Folgeströme und schließlich die Ausbeute an Octen berechnet. Dazu wurde ein kommerziell erhältliches Programm benutzt, welches unter dem Namen aspen plus von aspentech erhältlich ist.

### Erfindungsgemäßes Beispiel

Es wurde eine Verfahren simuliert, bei dem der Ausgangsstrom in einer 1. Stufe oligomerisiert wird und Oligomere abgetrennt werden (Verfahrensschritte a, b und c), aus dem Restrom iso-Butan destillativ abgetrennt wird (Verfahrensschritt d) und der danach erhaltene, iso-Butanarme Strom erneut oligomerisiert wird (Verfahrensschritt e), die Oligomerisierung in e) erfolgt hier zweistufig. Die bei den Oligomerisierungen erhaltenen Oligomere werden einer gemeinsamen Aufarbeitung zugeführt, bei der die Oligomeren in Octen, Dodecen und höhere Oligomere (C16 und höhere Oligomere, zusammenfassen als C16+ bezeichnet) aufgetrennt werden.

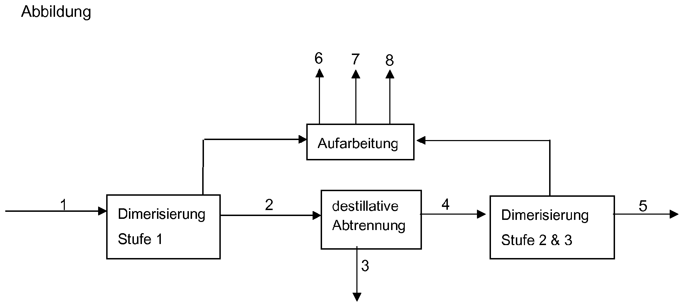

Dieses Verfahren ist in der Abbildung wiedergegeben. Nachstehend wird die berechnete Gesamtmenge und Zusammensetzung der einzelnen Ströme angegeben. Die Nummerierung der Ströme entspricht der Nummerierung in der Abbildung. Die Gesamtmenge ist in Tonnen/Stunde angegeben, Prozente sind Gewichtsprozente.

| Strom 1 | |
|---|---|
| Gesamtmenge | 52,5 t/h |
| n-Butan | 11,6 % |
| iso-Butan | 36,0 % |
| 1-Buten | 22,7 % |
| 2-Buten | 29,3 % |
| Verunreinigungen | 0,4 % |

| Strom 2 | |
|---|---|
| Gesamtmenge | 39,7 t/h |
| n-Butan | 15,3 % |
| iso-Butan | 47,6 % |
| 1-Buten | 2,5 % |
| 2-Buten | 34,1 % |
| Verunreinigungen | 0,5 % |

| Strom 3 | |
|---|---|
| Gesamtmenge | 20,2 t/h |
| n-Butan | 3,6 % |
| iso-Butan | 88,7 % |
| 1-Buten | 3,4 % |
| 2-Buten | 4,1 % |
| Verunreinigungen | 0,2 % |

| Strom 4 | |
|---|---|
| Gesamtmenge | 19,4 t/h |
| n-Butan | 27,6 % |
| iso-Butan | 5,0 % |
| 1-Buten | 1,7 % |
| 2-Buten | 65,5 % |
| Verunreinigungen | 0,2 % |

| Strom 5 | |
|---|---|
| Gesamtmenge | 7,0 t/h |
| n-Butan | 76,9 % |
| iso-Butan | 13,9 % |
| 1-Buten | 0,5 % |
| 2-Buten | 8,3 % |
| Verunreinigungen | 0,4 % |

Strom 6 (mehr als 99,9 % Octen): 20,2 t/h
Strom 7 (mehr als 99,9 % Dodecen): 3,9 t/h
Strom 8 (mehr als 99,9 % C16+): 1,1 t/h

### Vergleichsbeispiel

Zum Vergleich wurde ein Verfahren berechnet, bei dem keine destillative Abtrennung des iso-Butans erfolgt. In der Abbildung entfällt daher Strom 3 und Ströme 2 und 4 werden identisch.

| Strom 1 | |
|---|---|
| Gesamtmenge | 52,5 t/h |
| n-Butan | 11,6 % |
| iso-Butan | 36,0 % |
| 1-Buten | 22,7 % |
| 2-Buten | 29,3 % |
| Verunreinigungen | 0,4 % |

| Strom 2 = Strom 4 | |
|---|---|
| Gesamtmenge | 39,5 t/h |
| n-Butan | 15,4 % |
| iso-Butan | 47,8 % |
| 1-Buten | 2,5 % |
| 2-Buten | 33,9 % |
| Verunreinigungen | 0,4 % |

| Strom 5 | |
|---|---|
| Gesamtmenge | 28,7 t/h |
| n-Butan | 21,2 % |
| iso-Butan | 65,8 % |
| 1-Buten | 0,7 % |
| 2-Buten | 12,0 % |
| Verunreinigungen | 0,3 % |

Strom 6 (mehr als 99,9 % Octen): 18,6 t/h
Strom 7 (mehr als 99,9 % Dodecen): 4,0 t/h
Strom 8 (mehr als 99,9 % C16+): 1,2 t/h

Mit destillativer Abtrennung kann daher eine Ausbeute-Erhöhung von 8.6% Octen erreicht werden (siehe Strom 6 im erfindungsgemäßen Beispiel mit 20,2 t/h).

## Patentansprüche

1. Verfahren zur Herstellung von Oligomeren durch kontinuierliche Oligomerisierung von Butenen, **dadurch gekennzeichnet, dass**
a) ein Ausgangsstrom, welcher 1-Buten und 2-Buten in einer Gesamtkonzentration von 10 bis 70 Gew. % und 10 bis 60 Gew. % iso-Butan enthält, verwendet wird (im Nachfolgenden Ausgangsstrom 1 genannt)
b) dieser Ausgangsstrom 1 umgesetzt wird bis mehr als 60 Gew. % des im Ausgangsstrom 1 enthaltenen 1-Butens, aber weniger als 50 Gew. % des im Ausgangsstrom 1 enthaltenen 2-Butens in Oligomere überführt sind
c) die in b) erhaltenen Oligomere abgetrennt und gegebenenfalls einer weiteren Aufarbeitung zugeführt werden und der verbleibende Reststrom einer destillativen Aufarbeitung zugeführt wird
d) aus dem Reststrom iso-Butan destillativ abgetrennt wird
e) der nach der destillativen Aufarbeitung d) erhaltene, an iso-Butan abgereicherte Strom (im Nachfolgenden Ausgangsstrom 2 genannt) zu Oligomeren umgesetzt wird.

2. Verfahren gemäß Anspruch 1, **dadurch gekennzeichnet, dass** der Ausgangsstrom 1-Buten und 2-Buten in einer Gesamtkonzentration von 30 bis 60 Gew. % enthält.

3. Verfahren gemäß Anspruch 2, **dadurch gekennzeichnet, dass** Ausgangsstrom 1 5 bis 30 Gew. % 1-Buten und 20 bis 40 Gew. % 2-Buten enthält.

4. Verfahren gemäß einem der Ansprüche 1 bis 3, **dadurch gekennzeichnet, dass** Ausgangsstrom 1 30 bis 50 Gew. % iso-Butan enthält.

5. Verfahren gemäß einem der Ansprüche 1 bis 4, **dadurch gekennzeichnet, dass** der Ausgangsstrom 1 in Verfahrensschritt b) umgesetzt wird bis mehr als 80 Gew. % des in Ausgangstrom 1 enthaltenen 1-Butens, aber weniger als 50 Gew. % des 2-Butens in Oligomere überführt sind.

6. Verfahren gemäß einem der Ansprüche 1 bis 5, **dadurch gekennzeichnet, dass** die Gesamtmenge des Restroms 5 bis 95 Gew. % des Ausgangsstroms 1 beträgt.

7. Verfahren gemäß einem der Ansprüche 1 bis 6, **dadurch gekennzeichnet, dass** der Reststrom aus
30 bis 40 Gew. % 2-Buten
1 bis 10 Gew. % 1-Buten
40 bis 55 Gew. % iso-Butan
10 bis 20 Gew. % n-Butan
0 bis 5 Gew. % sonstigen Bestandteilen
besteht.

8. Verfahren gemäß einem der Ansprüche 1 bis 7, **dadurch gekennzeichnet, dass** es sich in Verfahrensschritt d) nicht um eine Extraktiv-Destillation handelt.

9. Verfahren gemäß einem der Ansprüche 1 bis 8, **dadurch gekennzeichnet, dass** in Verfahrensschritt d) aus dem Reststrom mehr als 80 Gew. % des iso-Butans abgetrennt werden.

10. Verfahren gemäß einem der Ansprüche 1 bis 9, **dadurch gekennzeichnet, dass** der nach Verfahrensschritt d) erhaltene Ausgangsstrom 2 aus
60 bis 70 Gew. % 2-Buten
0 bis 5 Gew. % 1-Buten
1 bis 10 Gew. % iso-Butan
20 bis 40 Gew. % n-Butan
0 bis 2 Gew. % sonstigen Bestandteilen
besteht.

11. Verfahren gemäß einem der Ansprüche 1 bis 10, **dadurch gekennzeichnet, dass** die Oligomeren aus der Oligomerisierung b) und e) einer gemeinsamen Aufarbeitung zugeführt werden.

12. Verfahren gemäß einem der Ansprüche 1 bis 11, **dadurch gekennzeichnet, dass** das im Ausgangstrom 1 enthaltene 1- und 2-Buten insgesamt zu mehr als 90 Gew. % in Oligomere überführt werden.

13. Verfahren gemäß einem der Ansprüche 1 bis 12, **dadurch gekennzeichnet, dass** es sich bei den insgesamt erhaltenen Oligomeren um ein Gemisch aus
70 bis 100 Gew.% Octen
0 bis 30 Gew. % Dodecen
0 bis 20 Gew. % höhere Oligomere
handelt.

## Claims

1. A process for preparing oligomers by continuous oligomerization of butenes, wherein
a) a feed stream comprising 1-butene and 2-butene in a total concentration of from 10 to 70% by weight and from 10 to 60% by weight of isobutane is used (hereinafter referred to as feed stream 1),
b) this feed stream 1 is reacted until more than 60% by weight of the 1-butene comprised in the feed stream 1 but less than 50% by weight of the 2-butene comprised in feed stream 1 have been converted into oligomers,
c) the oligomers obtained in b) are separated off and optionally passed to a further work-up and the remaining residual stream is fed to work-up by distillation,
d) isobutane is separated off by distillation from the residual stream,
e) the isobutane-depleted stream obtained after the work-up by distillation d) (hereinafter referred to as feed stream 2) is reacted to form oligomers.

2. The process according to claim 1, wherein the feed stream comprises 1-butene and 2-butene in a total concentration of from 30 to 60% by weight.

3. The process according to claim 2, wherein feed stream 1 comprises from 5 to 30% by weight of 1-butene and from 20 to 40% by weight of 2-butene.

4. The process according to any of claims 1 to 3, wherein feed stream 1 comprises from 30 to 50% by weight of isobutane.

5. The process according to any of claims 1 to 4, wherein the feed stream 1 is reacted in process step b) until more than 80% by weight of the 1-butene comprised in feed stream 1 but less than 50% by weight of the 2-butene are converted into oligomers.

6. The process according to any of claims 1 to 5, wherein the total amount of the residual stream is from 5 to 95% by weight of feed stream 1.

7. The process according to any of claims 1 to 6, wherein the residual stream consists of
from 30 to 40% by weight of 2-butene
from 1 to 10% by weight of 1-butene
from 40 to 55% by weight of isobutane
from 10 to 20% by weight of n-butane
from 0 to 5% by weight of other constituents.

8. The process according to any of claims 1 to 7, wherein process step d) is not an extractive distillation.

9. The process according to any of claims 1 to 8, wherein more than 80% by weight of the isobutane are separated off from the residual stream in process step d).

10. The process according to any of claims 1 to 9, wherein the feed stream 2 obtained after process step d) consists of
from 60 to 70% by weight of 2-butene
from 0 to 5% by weight of 1-butene
from 1 to 10% by weight of isobutane
from 20 to 40% by weight of n-butane
from 0 to 2% by weight of other constituents.

11. The process according to any of claims 1 to 10, wherein the oligomers from the oligomerizations b) and e) are passed to a joint work-up.

12. The process according to any of claims 1 to 11, wherein the total 1- and 2-butene comprised in feed stream 1 are converted to an extent of more than 90% by weight into oligomers.

13. The process according to any of claims 1 to 12, wherein the total oligomers obtained are a mixture of
from 70 to 100% by weight of octene
from 0 to 30% by weight of dodecene
from 0 to 20% by weight of higher oligomers.

## Revendications

1. Procédé pour la préparation d'oligomères par oligomérisation continue de butènes, **caractérisé en ce que**
a) un flux de départ, qui contient du 1-butène et du 2-butène en une concentration totale de 10 à 70% en poids et 10 à 60% en poids d'isobutane, est utilisé (appelé flux de départ 1 dans la suite)
b) ce flux de départ 1 est transformé jusqu'à ce que plus de 60% en poids du 1-butène contenu dans le flux de départ 1, mais moins de 50% en poids du 2-butène contenu dans le flux de départ 1 soient transformés en oligomère,
c) les oligomères obtenus dans b) sont séparés et le cas échéant introduits dans un traitement ultérieur et le flux résiduel qui reste est introduit dans un traitement par distillation,
d) l'isobutane est séparé par distillation du flux résiduel,
e) le flux obtenu après le traitement par distillation d) appauvri en isobutane (appelé flux de départ 2 dans la suite) est transformé en oligomères.

2. Procédé selon la revendication 1, **caractérisé en ce que** le flux de départ contient du 1-butène et du 2-butène en une concentration totale de 30 à 60% en poids.

3. Procédé selon la revendication 2, **caractérisé en ce que** le flux de départ 1 contient 5 à 30% en poids de 1-butène et 20 à 40% en poids de 2-butène.

4. Procédé selon l'une quelconque des revendications 1 à 3, **caractérisé en ce que** le flux de départ 1 contient 30 à 50% en poids d'isobutane.

5. Procédé selon l'une quelconque des revendications 1 à 4, **caractérisé en ce que** le flux de départ 1 est transformé dans l'étape de procédé b) jusqu'à ce que plus de 80% en poids du 1-butène contenu dans le flux de départ 1, mais moins de 50% en poids du 2-butène soient transformés en oligomères.

6. Procédé selon l'une quelconque des revendications 1 à 5, **caractérisé en ce que** la quantité totale du flux résiduel représente 5 à 95% en poids du flux de départ 1.

7. Procédé selon l'une quelconque des revendications 1 à 6, **caractérisé en ce que** le flux résiduel est constitué par
30 à 40% en poids de 2-butène
1 à 10% en poids de 1-butène
40 à 55% en poids d'isobutane
10 à 20% en poids de n-butane
0 à 5% en poids d'autres constituants.

8. Procédé selon l'une quelconque des revendications 1 à 7, **caractérisé en ce que**, dans l'étape de procédé d), il ne s'agit pas d'une distillation extractive.

9. Procédé selon l'une quelconque des revendications 1 à 8, **caractérisé en ce que**, dans l'étape de procédé d), plus de 80% en poids de l'isobutane sont séparés du flux résiduel.

10. Procédé selon l'une quelconque des revendications 1 à 9, **caractérisé en ce que** le flux de départ 2 obtenu après l'étape de procédé d) est constitué par
60 à 70% en poids de 2-butène
0 à 5% en poids de 1-butène
1 à 10% en poids d'isobutane
20 à 40% en poids de n-butane
0 à 2% en poids d'autres constituants.

11. Procédé selon l'une quelconque des revendications 1 à 10, **caractérisé en ce que** les oligomères provenant de l'oligomérisation b) et e) sont introduits dans un traitement commun.

12. Procédé selon l'une quelconque des revendications 1 à 11, **caractérisé en ce que** le 1-butène et le 2-butène contenus dans le flux de départ 1 sont transformés au total à raison de plus de 90% en poids en oligomères.

13. Procédé selon l'une quelconque des revendications 1 à 12, **caractérisé en ce qu'**il s'agit, pour les oligomères obtenus au total, d'un mélange de
70 à 100% en poids d'octène
0 à 30% en poids de dodécène
0 à 20% en poids d'oligomères supérieurs.
